Europäisches Patentamt

European Patent Office

Office européen des brevets

(11) Publication number: **0 375 174**
**A2**

# (12) EUROPEAN PATENT APPLICATION

(21) Application number: **89312326.5**

(22) Date of filing: **28.11.89**

(51) Int. Cl.5: **C08B 37/00, A61K 31/72**

(30) Priority: **28.11.88 JP 300293/88**

(43) Date of publication of application:
**27.06.90 Bulletin 90/26**

(84) Designated Contracting States:
**DE FR GB**

(71) Applicant: **AJINOMOTO CO., INC.**
**5-8, Kyobashi 1-chome, Chuo-ku**
**Tokyo 104(JP)**

Applicant: **FUJIREBIO INC.**
**6-7, Shimoochiai 4-chome Shinjuku-ku**
**Tokyo 161(JP)**

(72) Inventor: **Yasuda, Naohiko c/o Central**
**Research Laboratories**
**Ajinomoto Co., Inc. No. 1-1 Suzuki-cho**
**Kawasaki-ku Kawasaki-shi**
**Kanagawa-ken(JP)**
Inventor: **Shioya, Shigeru c/o Central**
**Research Laboratories**
**Ajinomoto Co., Inc. No. 1-1 Suzuki-cho**
**Kawasaki-ku Kawasaki-shi**
**Kanagawa-ken(JP)**
Inventor: **Kaneko, Yutaro**
**c/o Ajinomoto Co., Inc. No. 5-8 Kyobashi**
**1-chome**
**Chuo-ku Tokyo(JP)**
Inventor: **Uryu, Toshiyuki**
**No. 3-5-26, Aoi**
**Adachi-ku Tokyo(JP)**
Inventor: **Yamamoto, Naoki**
**No. 1-7-12, Higashiobayama-cho**
**Ube-shi Yamaguchi-ken(JP)**
Inventor: **Matsuzaki, Kei**
**No. 4-10-10 Naritahigashi**
**Suginami-ku Tokyo(JP)**

(74) Representative: **Armitage, Ian Michael et al**
**MEWBURN ELLIS 2 Cursitor Street**
**London EC4A 1BQ(GB)**

(54) **Lentinan and curdlan sulfates for anti-retroviral use.**

(57) Lentinan and curdlan sulfates are produced at Mw 20,000-330,000 and S-content 14.5% ± 2.5% for anti-HIV use.

The process for their preparation preferably involves pre-drying of lentinan or curdlan to a water content of 2% or less. The curdlan may have been pretreated by stepwise conversion from a polar or aqueous solution to a less polar or non-aqueous solution.

EP 0 375 174 A2

## LENTINAN AND CURDLAN SULFATES FOR ANTI-RETROVIRAL USE

The present invention relates to novel anti-viral agents containing a modified $\beta$-(1→3)-glucan.

More particularly it relates to such compounds which have improved anti-retroviral, especially anti-reverse transcription (anti-RT) activity, and are therefore useful against e.g. human immunodeficiency virus (hereinafter referred to as "HIV").

DE OS 2110488 indicates anti-bacterial and antiviral (but not specifically anti-RT) uses for sulfated polymers, such as pentosan sulfate, dextran sulfate, heparin etc. Dextran sulfate is mentioned, with a MW of 50Kd or 500Kd. However, the mechanism of anti-RT activity is completely different from anti-viral activity for other types of virus, so that there is no teaching here of anti-RT activity, or how it may be optimized.

Hoffman et al, Carbohydrate Polymers, Vol.2, No.2, 1982, pp.115-121, dealt with the anti-coagulant effect of sulfated polysaccharides, including curdlan sulfate, but not the anti-RT effect.

Hatanaka et al, J. of Med. Chem., Vol. 30, No.5, pp.810-814, likewise concentrated anti-coagulant activity. It acknowledges (page 812) that anticoagulant activity of dextran sulfate increases with MW (but becomes more toxic).

It is known that inorganic ion-containing organic high polymer compounds can have an effect indicating prevention and remedy of retroviral infectious diseases (Japanese Patent Application Laid-Open No. 62-215529) and, in particular, sulfates of polysaccharides are also known for this effect (Japanese Patent Application Laid-Open No. 63-45223). However, there are many aspects of those compounds which are unclear, for example the structure which provides anti-RT activity, and that which provides blood-coagulating activity, the latter being undesirable in the anti-RT use of the compounds. Accordingly, practical sulfated polysaccharides for anti-RT use have not been established.

Yamamoto et al (Arch. of Aids Res. Vol.1, No. 1, 1987, pp. 45-56) discloses the anti-RT effect of a variety of sulfated polysaccharides. Curdlan and lentinan sulfates are mentioned, along with dextran sulfates and others. There is a suggestion that the degree of sulfation is related to the degree of RT inhibitory activity. However, no indications are given as to the appropriate molecular weights (MW). Only dextran sulfate has its MW identified: two species at 34 Kd and 7 Kd, both having anti-RT activity. Lentinan sulfate, but not curdlan sulfate, is tested for anti-HIV activity, but the results indicate that dextran sulfate is generally superior to lentinan sulfate.

EP 232744A discloses particularly the anti-RT use of dextran sulfates of MW 5Kd, 8Kd and 500Kd, as well as pentosan polysulfate of 5Kd. Lentinan and curdlan sulfates are not tested, nor even mentioned.

EP 240098A discloses polysaccharide sulfates for RT inhibition, and that RT inhibition increases with sulfur content. Lentinan sulfate is mentioned among the numerous polysaccharides suggested, but not with any particular emphasis, and the disclosure contains little information as to MW. For dextran sulfate a MW of 3,000-8,000 is particularly recommended.

As regards the methods for sulfating polysaccharides and isolating them, a detailed investigation has already been made on dextran sulfate, a sulfate of $\alpha$-(1→6)-glucan, (Carbohydrate Research, 21 (1972), 420-426), but a detailed investigation on $\beta$-(1→3)-glucan has not been made up to the present.

Our own application EP 298706A, (relevant only under Art 54(3) EPC), discloses a variety of glycan sulfates for anti-HIV use. Among the many such compounds mentioned, lentinan and curdlan derivatives are suggested, and were prepared and tested. However, the disclosure there is not specific as to sulfur content and molecular weight.

In contrast, the present invention provides novel processes for preparing such lentinan and curdlan derivatives in which it is possible to control the MW and sulfur content. In another aspect, the present invention provides a compound for use as an anti-retroviral agent, said compound comprising a sulfated derivative of lentinan or curdlan, or a salt thereof, having a sulfur content of 14.5% ± 2.5% and a weight average molecular weight by gel permeation chromatography (GPC) of about 20,000 or more.

As the data herein show, lentinan and curdlan sulfates within the specified ranges of sulfur content and MW have remarkably better anti-HIV activity and lower anti- coagulant activity than dextran sulfates; a result which is entirely surprising in view of the prior art, and points to these compounds, from among the many sulfated polysaccharides mentioned in the prior art, as being particularly suitable for anti-retroviral, and especially anti-HIV treatment and prophylaxis.

The following preferred methods may be used for preparing the compounds hereof.

The curdlan sulfate can be prepared from known curdlan as the raw material. As curdlan has a low solubility, it is desirable that curdlan is previously activated so as to elevate the solubility. The activating treatment to be applied to curdlan for the said purpose may be effected by varying stepwise a suspension of curdlan in a more polar or aqueous solvent to that in a less polar or non-aqueous solvent. The curdlan is preferably previously dried to lower the water content thereof to 2% or less for the purpose of effecting the reaction quantitatively and stably. Dimethylsulfoxide (hereinafter referred to as "DMSO") and a sulfating agent such as piperidine sulfate are also fully dried and then used in the sulfation reaction. As well as sulfating the curdlan, this process can also have the effect of lowering the MW to the desired level. As curdlan is difficult to dissolve in DMSO, it is preferred to stir it in DMSO for several hours to overnight at room temperature, or for several hours at 60°C to 90°C, for complete dissolution. Next, the resulting solution is reacted with a sulfating agent such as piperidine sulfate, which is used in an amount of from 1 to 3 equivalents per glucose residue, at a reaction temperature of 60°C to 90°C and a reaction time of 1 hour to 24 hours. Stronger conditions than the above, for example, 3 equivalents or more piperidine sulfate, and/or a temperature higher than 90°C and/or a reaction time longer than 24 hours may often be accompanied by a side-reaction with coloration, excessive lowering of the MW, or release of sulfate radical.

The reaction mixture may be neutralized with, for example, sodium hydroxide or sodium bicarbonate, and then subjected to dialysis so as to isolate the sulfated curdlan. However, as dialysis requires a long time, and removal of impurities may be insufficient by this method, it is preferred that the product is isolated from the reaction solution in the form of a precipitate and then separated from DMSO and other components. For precipitation of a high polymer substance the addition of an organic solvent to the reaction mixture is commonly used. However, the product of the invention does not readily precipitate with only an organic solvent. It has been surprisingly found that addition of a salt together with an organic solvent is effective for efficient precipitation of the product in the form of e.g. its sodium salt. The salt to be used for the purpose may be an inorganic salt such as sodium chloride. However, as the salt is an impurity it is desirably an organic solvent-soluble salt, for example, sodium acetate, which can be washed and removed with an organic solvent, thereby enhancing the purity of the product.

The precipitate obtained, which comprises the product and the salt, is washed several times with an organic solvent and then dissolved in water. The washing may be effected batchwise by repeated washing and centrifugal treatment. However, use of a ceramic membrane is more preferred as the washing can be effected continuously.

The aqueous solution containing the product can be desalted by passing through an ultrafiltration membrane, for which a ceramic membrane can also be used. The resulting aqueous solution containing only the product may be directly freeze-dried. Where the product is obtained in the form of a powder, an appropriate amount of an organic solvent-soluble salt may be added to the aqueous solution, and the intended product can be precipitated with an organic solvent.

The lentinan sulfate derivatives can be obtained from known lentinan as the raw material. In the sulfation of lentinan, as with curdlan, the lentinan is preferably previously dried to lower the water content therein to 2% or less, and DMSO and a sulfating agent such as piperidine sulfate are also fully dried and then used in the sulfation reaction. As lentinan is difficult to dissolve in DMSO, it is important that lentinan is stirred in DMSO for several hours at room temperature for complete dissolution.

For isolation of the product from the reaction solution, the same process as that used for obtaining the product for curdlan may also be used.

The present invention will be explained in detail by way of the following examples.

Example 1: Preparation of modified β-(1→3)-glucan from curdlan:

1g of curdlan was suspended in 50% acetone for 24 hours, then in 75% acetone for 24 hours, then in 100% acetone for 24 hours, and then in 100% ether for 24 hours. Afterwards, the precipitate formed was dried overnight under reduced pressure in the presence of phosphorus pentoxide. 1g of the thus activated dry curdlan was added to 100 ml of previously dried DMSO with Molecular Sieve 4A and stirred overnight at room temperature, whereby the curdlan was dissolved in the DMSO. 2.5g of piperidine sulfate was added and heated to 85°C, and the reaction was continued for 1 hour under these conditions. After completion of the reaction, the reaction mixture was cooled with water and 500 ml of 5% solution of sodium acetate in methanol was added, whereupon the product precipitated out. The resulting precipitate was washed twice with 100 ml of methanol and then dissolved in 100 ml of water, and the resulting solution was de-salted through an ultrafiltration membrane (Amicon Co. hollow fibre) having a cut-off for compounds at molecular weight of 10,000. To 100ml of aqueous solution of the thus de-salted product was added 400 ml of a 5% solution of sodium acetate in methanol, whereby the product precipitated out. The precipitate was washed twice with 100ml of methanol and then dried under reduced pressure to obtain 1.7g of the modified β-(1→3)-glucan (Compound No. CS-1).
S-content (Elementary Analysis): 14.4%

3

Weight Average Molecular Weight (Gel Filtration Method): 113,000

In the same manner as above, other modified $\beta$-(1→3)-glucans, Compounds Nos. CS-2 to CS-11 were obtained.

Example 2: Preparation of modified $\beta$-(1→3)-glucan from lentinan:

1g of lentinan, dried overnight under reduced pressure in the presence of phosphorus pentoxide, was added to 100 ml of previously dried DMSO with Molecular Sieve 4A and stirred for 2 hours at room temperature to dissolve it. 2.3g of piperidine sulfate was added, the whole was heated up to 85°C and then reacted for 1 hour under these conditions. After completion of the reaction, the reaction mixture was cooled with water, and 50 ml of 30% sodium acetate and then 600 ml of acetone were added to precipitate the product. The resulting precipitate was washed several times with 200 ml of acetone and then dissolved in 100 ml of water and then de-salted through 0.14 $\mu$m ceramic membrane. To 100 ml of aqueous solution of the thus de-salted product was added 400 ml of a 5% solution of sodium acetate in methanol, whereby the product precipitated out. The precipitate was washed twice with 100 ml of methanol and then dried under reduced pressure to obtain 1.8 g of the modified $\beta$-(1→3)-glucan (Compound No. LS-1).

S-content (Elementary Analysis): 14.2%

Weight Average Molecular Weight (Gel Filtration Method): 158,000

In the same manner as above, other modified $\beta$-(1→3)-glucans, Compounds Nos. LS-2 to LS-8 were obtained.

Example 3: Determination of anti-HIV activity and anti-coagulating activity of modified $\beta$-(1→3)-glucans:

MT-4 cells were infected with HTLV-III at moi = 0.002. The specimen to be examined was diluted with medium to a predetermined concentration and mixed with the infected cells in a ratio of 1:1, and 30 x 10⁴ cells/ml were incubated. 6 days after incubation, the number of the living cells and the antigen-positive ratio were determined.

The specimen to be examined was blended with 50 parts by volume of rat plasma and 1 part by volume of a solution of the test substance as stepwise diluted with a physiological salt solution, and the activated part thromboplastin time (APTT) of the specimen was determined. The concentration of the test substance to double the APTT was obtained, and this was compared with the concentration of sodium heparin solution (whose titer of anti-coagulating activity is known) to double the APTT of the control specimen, whereby the anti-coagulating activity of the test substance was represented as the titer of sodium heparin in unit/mg. The results were summarized in the following Table.

From the table it can be seen that the anti-viral agent of the present invention which comprises modified $\beta$-(1→3)-glucan obtained from curdlan or lentinan has a stronger anti-HIV activity and a weaker anti-coagulating activity than conventional dextran sulfates and it is therefore a more useful anti-viral agent.

4

| | S-content (%) | Weight Average Molecular Weight by GPC ($\times 10^4$) | Anti-Coagulating Activity (u/mg) | Anti-HIV Activity | | | |
|---|---|---|---|---|---|---|---|
| | | | | 3.3 ug/ml | | 10 ug/ml | |
| | | | | Percentage of Living Cells (%) | Percentage of Positive Antigen (%) | Percentage of Living Cells (%) | Percentage of Positive Antigen (%) |
| LS-1 | 14.2 | 15.8 | 9.0 | 107 | 1 | 100 | 1 |
| LS-2 | 15.2 | 7.3 | 15.0 | 95 | 1 | 84 | 1 |
| LS-3 | 12.5 | 14.9 | 8.9 | 121 | n.a. | 103 | n.a. |
| LS-4 | 13.4 | 11.6 | 11.2 | 90 | 1 | 99 | 1 |
| LS-5 | 15.2 | 30.5 | 11.1 | 89 | 1 | 103 | 1 |
| LS-6 | 12.8 | 26.3 | 11.2 | 77 | 1 | 101 | 1 |
| LS-7 | 11.7 | 11.4 | n.a. | 3.2 | 90 | 46 | 90 |
| LS-8 | 10.7 | 17.4 | 10.6 | 1.6 | 90 | 3.2 | 90 |
| CS-1 | 14.4 | 11.3 | 10.9 | 104 | 1 | 102 | 1 |
| CS-2 | 12.4 | 33.0 | 16.5 | 112 | 1 | 103 | 1 |
| CS-3 | 14.0 | 4.5 | 9.5 | 113 | 1 | 99 | 1 |
| CS-4 | 13.1 | 10.2 | n.a. | 107 | 1 | 93 | 1 |
| CS-5 | 11.7 | 13.1 | n.a. | 0 | 90 | 4.5 | 90 |
| CS-6 | 16.2 | 2.7 | 15.3 | 100 | 1 | 103 | 1 |
| CS-7 | 14.7 | 15.2 | 16.2 | 115 | 1 | 114 | 1 |
| CS-8 | 12.8 | 22.9 | 12.7 | 104 | 1 | 110 | 1 |
| CS-9 | 16.8 | 6.5 | 11.2 | 94 | 1 | 97 | 1 |
| CS-10 | 14.9 | 2.0 | 5.8 | 0.6 | 90 | 0.6 | 90 |
| CS-11 | 14.8 | 4.8 | 10.6 | 83 | 10 | 92 | 1 |
| CS-12 | 14.5 | 3.7 | n.a. | 76 | 21 | 99 | 1 |
| CS-13 | 10.7 | 4.2 | 1.2 | 1.9 | 90 | 0.6 | 90 |
| CS-14 | 15.0 | 6.0 | 14.9 | 101 | 1 | 109.0 | 1 |
| CS-15 | 10.6 | 27.9 | 2.0 | 1.1 | 90 | 1.1 | 90 |
| CS-16 | 11.0 | 12.3 | 2.7 | 1.6 | 90 | 2.1 | 90 |
| CS-17 | 12.5 | 19.9 | 9.8 | 94 | 1 | 107 | 1 |
| CS-18 | 11.8 | 8.6 | 3.3 | 2.7 | 90 | 2.7 | 90 |
| CS-19 | 11.7 | 23.0 | 12.3 | 11 | 90 | 4.5 | 90 |
| CS-20 | 11.8 | 11.6 | 3.9 | 1.7 | 90 | 2.3 | 90 |
| CS-21 | 15.1 | 7.2 | 15.7 | 106 | 1 | 94 | 1 |
| CS-22 | 14.6 | 5.7 | 10.1 | 98 | 1 | 110 | 1 |
| CS-23 | 14.7 | 10.1 | 9.8 | 102 | 1 | 110 | 1 |
| CS-24 | 14.1 | 10.6 | 9.8 | 96 | 1 | 94 | 1 |
| CS-25 | 15.4 | 4.6 | 12.6 | 99 | 1 | 110 | 1 |
| CS-26 | 16.0 | 4.8 | 12.2 | 105 | 1 | 110 | 1 |
| CS-27 | 13.7 | 15.6 | 14.7 | 104 | 1 | 105 | 1 |
| CS-28 | 14.8 | 4.4 | 10.6 | 103 | 1 | 106 | 1 |
| CS-29 | 13.8 | 15.1 | 11.1 | 121 | 1 | 120 | 1 |
| CS-30 | 13.6 | 16.3 | 10.6 | 107 | 1 | 104 | 1 |

| | S-content (%) | Weight Average Molecular Weight by GPC (x10$^4$) | Anti-Coagulating Activity (u/mg) | Anti-HIV Activity | | | |
|---|---|---|---|---|---|---|---|
| | | | | 3.3 ug/ml | | 10 ug/ml | |
| | | | | Percentage of Living Cells (%) | Percentage of Positive Antigen (%) | Percentage of Living Cells (%) | Percentage of Positive Antigen (%) |
| DS-1 | 17.9 | 1.7 | 38.6 | 2.1 | 90 | 93.0 | 1 |
| DS-2 | 17.9 | 12.2 | 71.9 | 1.5 | 90 | 93.0 | 1 |
| DS-3 | 17.9 | 2.3 | 33.0 | 3.1 | 90 | 93.0 | 1 |
| DS-4 | 17.5 | 1.6 | 37.2 | 1.0 | 90 | 70.0 | 90 |
| DS-5 | 17.4 | 3.2 | 54.2 | 92.2 | 2 | 85.0 | 1 |

## Claims

1. A compound for use as an anti-retroviral agent, said compound comprising a sulfated derivative of lentinan or curdlan, or a salt thereof, having a sulfur content of 14.5% ± 2.5% and a weight average molecular weight by gel permeation chromatography (GPC) of about 20,000 or more.

2. A compound according to claim 1 wherein the MW is in the range 20,000 to 330,000 as measured by GPC.

3. The use of a compound of claim 1 or claim 2 in the preparation of a pharmaceutical composition for the treatment or prophylaxis of retroviral infection.

4. A process for preparing a curdlan or lentinan sulfate, or a salt thereof, of claim 1 or claim 2 by sulfating curdlan or lentinan, characterised in that the sulfating process takes place in substantially anhydrous solution, and the curdlan or lentinan is subjected to drying before sulfation.

5. A process according to claim 4 wherein the curdlan or lentinan is subjected to drying to a water content of 2% or less before being dissolved in a solvent for the sulfation process.

6. A process according to claim 5 wherein the solvent for sulfation is substantially anhydrous dimethyl sulfoxide.

7. A process according to any one of claims 4, 5 and 6 wherein the drying is effected under reduced pressure in the presence of phosphorus pentoxide.

8. A process according to any one of claims 4 to 7, in which curdlan or lentinan having a water content of 2% or less is reacted with from 1 to 3 equivalents of a sulfating agent.

9. A process according to any one of claims 4 to 8 wherein the curdlan or lentinan is pretreated to make it more readily soluble, said pretreatment comprising suspending the curdlan or lentinan in a polar or aqueous solvent, and varying the solvent stepwise towards a less polar or non-aqueous solvent.

10. A process according to claim 9 wherein said less polar solvent is substantially anhydrous.

11. A process for preparing a curdlan or lentinan sulfate, or a salt thereof, of claim 1 or claim 2 by sulfating curdlan or lentinan, characterised in that the curdlan or lentinan is pretreated to make it more readily soluble, said pretreatment comprising suspending the curdlan or lentinan in a polar or aqueous solvent, and varying the solvent stepwise towards a less polar or non-aqueous solvent.

12. A process according to claim 11 wherein said less polar solvent is substantially anhydrous.

13. A process for preparing a curdlan or lentinan sulfate, or a salt thereof, of claim 1 or claim 2 by sulfating curdlan or lentinan, which process includes salting out and precipitating the curdlan or lentinan sulfate with a salt and an organic solvent, with concomitant neutralisation of the product.

14. A process according to claim 13 wherein said salt is soluble in an organic solvent.

15. A process according to claim 14 in which the salting out, neutralisation and precipitation are effected using sodium acetate in methanol.

16. A process for preparing a curdlan or lentinan sulfate, or a salt thereof, of claim 1 or claim 2 by sulfating curdlan or lentinan, which process includes purifying the curdlan or lentinan sulfate by washing and ultrafiltration using a ceramic membrane.

17. A process according to claim 16 wherein the ceramic membrane has a cut-off for compounds at about MW 10,000.

18. A process according to claim 16 wherein the ceramic membrane has a pore size of about 0.14 $\mu$m.